# EUROPEAN PATENT SPECIFICATION

(11) **EP 0 826 964 B1**
(45) Date of publication and mention of the grant of the patent: **22.01.2003**
(21) Application number: 97402023.2
(22) Date of filing: 29.08.1997
(51) Int. Cl.: G01N 33/68, C07K 14/31

(54) **Determination of protein**
Bestimmung von Proteinen
Dosage de protéine

(30) Priority: 30.08.1996 JP 24685996
(43) Date of publication of application: 04.03.1998
(73) Proprietor: ORIENTAL YEAST CO., LTD., Tokyo (JP)
(72) Inventor: Kihira, Yasunori, Ina-shi, Nagano-ken (JP); Matsuo, Yuhsi, Suita-shi, Osaka-fu (JP)
(74) Representative: Clisci, Serge

(56) References cited:
- EP-A- 0 550 771
- WO-A-95/10041
- PATENT ABSTRACTS OF JAPAN vol. 004, no. 115 (P-023), 16 August 1980 & JP 55 071945 A (ORIENTAL YEAST CO LTD), 30 May 1980

### SEQUENCE LISTING

(1) GENERAL INFORMATION:
   (i) APPLICANT:
      (A) NAME: ORIENTAL YEAST CO., LTD.
      (B) STREET: 6-10, Azusawa 3-chome, Itabashi-ku
      (C) CITY: Tokyo
      (E) COUNTRY: Japan
      (F) POSTAL CODE (ZIP): none
   (ii) TITLE OF INVENTION: DETERMINATION OF PROTEIN
   (iii) NUMBER OF SEQUENCES: 2
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: JP 8-246859
      (B) FILING DATE: 30-AUG-1996
(2) INFORMATION FOR SEQ ID NO: 1:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (v) FRAGMENT TYPE: N-terminal
   (xi) SEQUENCE DESCRIPTION: SEQ ID NO: 1:
   Met Lys Val Asp
   1
(2) INFORMATION FOR SEQ ID NO: 2:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (v) FRAGMENT TYPE: C-terminal
   (xi) SEQUENCE DESCRIPTION: SEQ ID NO: 2:
   Thr Gly Arg Arg Phe Thr Thr Ser
   1               5

## Description

### Technical Field to Which the Invention Belongs

The present invention relates to a system in which a target protein to be analyzed is determined for its molecular weight (type) and amount (amount of the protein) simultaneously and easily by use of a marker.

The present invention plays an important part in fields such as biochemistry, clinical chemistry and analytical chemistry.

### Prior Art

There is demand for the techniques of separating and analyzing a protein in a variety of biotechnology-related fields. In particular, SDS-polyacrylamide gel electrophoresis (SDS-PAGE) is generally and frequently used to analyze the purity of a protein sample and to estimate the molecular weight of a target protein. The density of a band is proportional to the amount of a charged protein in SDS-PAGE, so it is tentatively possible to estimate the concentration of a target protein in a sample by charging different lanes with a changing amount of a a protein of known amount for comparison of density.

However, a conventionally used marker protein was inappropriate for the estimation of the amount of the target protein because such protein was a mixture of various proteins with variability in stainability among their respective bands. Moreover, in cases where the mixing ratio of the respective marker proteins was to be regulated, all bands of the marker proteins were regulated to exhibit a constant density or a specific band was regulated to be darker as a mark. Therefore, it was necessary to charge and electrophorese different amounts of proteins on plural lanes in order to quantify a sample protein.

### Problem to Be Solved by the Invention

The object of the present invention is to solve the problem of the prior art. The present inventors developed a new marker and made the present invention with the aim of developing a novel measurement system in which a calibration protein curve can be prepared from a staining pattern of said marker in one lane after regulation of the ratio in amount of the respective marker proteins having different molecular weights.

### Means to Solve the Problem

The present invention was completed at last as a result of extensive research to achieve said object, and it relates to a method in which a target protein is determined for its molecular weight (type) and content (amount of the protein) simultaneously and very easily by simultaneous electrophoresis of the marker and a sample containing a target protein to be analyzed.

That is, the present invention embraces, as a fundamental technical concept, a method of determining the molecular weight (type) and/or the amount (content) of a target protein wherein a marker consisting of proteins having different molecular weights and being regulated at the same or different amounts is electrophoresed simultaneously with a sample containing a target protein to be analyzed (a mixture containing a single protein or several proteins) and then stained, and the densities of the bands are compared with one another visually or with a densitometer.

To be precise, the method for determining the molecular weight and/or the amount of a target protein, which is recommended here, comprises
(1) subjecting a target protein to electrophoresis simultaneously with a marker consisting of proteins having different molecular weights, the respective amounts of the proteins of the marker being different from one another,
(2) staining the target protein and the proteins of the marker, and
(3) comparing the bands induced in step (2) from one another by eye or with a densitometer,
whereby the proteins of the marker are IgG-binding artificial proteins each comprising one or several iterations of a unit comprising one or several domains selected from the group consisting of domains A, B, C, D and E of protein A.

### Brief Description of the Drawings

FIG. 1 is a photograph of a stained gel after SDS-polyacrylamide gel electrophoresis of a protein standard (molecular weight marker) and BSA.

FIG. 2 is a densitogram showing the result of the analysis of the protein standard lane in FIG. 1 in a densitometer.

FIG. 3 is a protein calibration curve prepared on the basis of the results in FIGS. 1 and 2.

FIG. 4 is a photograph of a stained gel after SDS-polyacrylamide gel electrophoresis of the protein standard charged in different amounts.

### Embodiments for Carrying Out the Invention

Hereinafter, the present invention is described in detail.

A marker consisting of proteins having different molecular weights and being regulated at the same or different amounts is widely used as the marker of the present invention. Preferable marker proteins are separated from one another in electrophoresis to form individual sharp zones with uniform and excellent stainability.

The marker in the present invention is dissolved in a suitable liquid, e.g., buffer and used for electrophoresis.

Such a preferable marker includes a molecular weight marker. Examples of marker proteins are proteins comprising each one or several domains selected from the group consisting of domains E, D, A, B and C of protein A. Proteins comprising each one or several domains selected from the group consisting of domains C₁, C₂ and C₃ of protein G and proteins comprising each one or several domains selected from the group of domains of other IgG-binding proteins (e.g. protein M) can be used similarly.

Such molecular weight marker proteins can be produced by genetic engineering means as described in Japanese Patent No. 2519136, and for example, the following marker can be obtained:

"A molecular weight marker consisting of at least two IgG-binding artificial proteins each comprising one or more iterations of a unit comprising one or more IgG-binding domains selected from the group consisting of domains A, B, C, D, and E of protein A"; the IgG-binding domains mean domains capable of bonding to IgG.

A molecular weight marker proteins each comprising pA(AB)ₙ (n = 1 to 6) comprises domains A and B of protein A as the unit as above-mentioned show a high water solubility, and contrasting with natural proteins, such artificial marker proteins each do not contain a modified protein (and thus each protein has a completely identical molecular weight), and therefore, the marker proteins of a mixture thereof at suitable amount-proportion can be separated from one another in SDS-PAGE to form individual sharp zones, bringing about individual clear bands in gel staining (e.g. dye staining and silver staining) after electrophoresis, and such a marker can be used advantageously in the present invention as a highly accurate molecular weight marker for electrophoresis.

The number of linked domains (domains A and B, or domain D, as a repeating unit) in each marker protein thus produced is as shown in Tables 1 and 2 below:

**Table 1**

| Marker Protein | Number of Domains A and B | Molecular Weight |
|---|---|---|
| PROT-ABI | 1 | 14,586 |
| PROT-ABII | 2 | 27,987 |
| PROT-ABIII | 3 | 41,389 |
| PROT-ABIV | 4 | 54,790 |
| PROT-ABV | 5 | 68,192 |
| PROT-ABVI | 6 | 81,593 |
| Structure of marker protein: | | |
| NH₂-MKVD-(domains A and B)ₙ-TGRRFTTS-COOH | | |
| n = Number of linked domains A and B | | |

**Table 2**

| Marker Protein | Number of Domain D | Molecular Weight |
|---|---|---|
| PROT-D₁ | 1 | 8,220 |
| PROT-D₂ | 2 | 15,256 |
| PROT-D₃ | 3 | 22,291 |
| PROT-D₄ | 4 | 29,327 |
| PROT-D₅ | 5 | 36,362 |
| Structure of marker protein: | | |
| NH ₂-MKVD-(domain D)ₙ-TGRRFTTS-COOH | | |
| n = Number of linked domain D | | |

The present molecular weight marker composed of a mixture of pA(AB)ₙ (n = 1 to 6) having domains A and B of protein A as a repeating unit overcomes the ununiform stainability of each marker protein band which is the disadvantage of a conventional molecular weigh marker composed of natural proteins, and the present molecular weight marker can be used as a scale for quantification of a protein by utilizing the uniform stainability of pA(AB)ₙ and thereby regulating the mixing ratio. That is, the mere regulation of the ratio in amount of constituent marker proteins does not suffice, and this technique has been made feasible for the first time by further using a group of proteins with uniform stainability and having different molecular weights.

In the present invention, the molecular weight and protein amount as desired can be arbitrarily regulated by selecting the type and mixing ratio of the constituent marker proteins. In addition, the necessary data for preparing a calibration curve are obtained by electrophoresing the molecular weight marker in only one lane. Because the amount of a protein is proportional to the density of its band, the amount of the protein can visually be estimated approximately or can be determined using a calibration curve and further accurately with a densitometer.

For the determination of a target protein in a sample according to the present invention, a molecular weight marker composed of proteins with different molecular weights and regulated at different amounts is prepared by mixing e.g. pA(AB)₁ to pA(AB)₆ in amounts of 1.0, 0.8, 0.6, 0.4, 0.2 and 0.1 µg respectively.

Therefore, it is essential that the measurement kit used for carrying out the present invention contains such a molecular weight marker. Said measurement kit may further contain a set of a buffer, a cloring agent, other necessary reagent etc. as required.

The molecular weight marker thus prepared is electrophoresed simultaneously with a sample containing a target protein to be analyzed (which can be analyzed whether it contains a single protein or a mixture of proteins) and then stained, and the band of each marker protein in the lane of the molecular weight marker is compared with the position and/or density of each band in the sample lane, whereby the molecular weight (type) and/or the amount (content or concentration) of the target protein is determined.

Therefore, the present invention is epoch-making in that it enables quantification of a protein in a sample by the density of the band and simultaneous estimation of its type by the position of the band.

Further, because this measurement can also be effected by eye or with a densitometer, the target protein can be measured or estimated visually though approximately in the case of hurry or with a densitometer for more accurate measurement, so the measurement can be effected as necessary.

Furthermore, the present invention is superior in that not only a single protein but also a mixture of various proteins can be determined all at once.

Hereinafter, the present invention is illustrated with reference to Example.

### Example 1

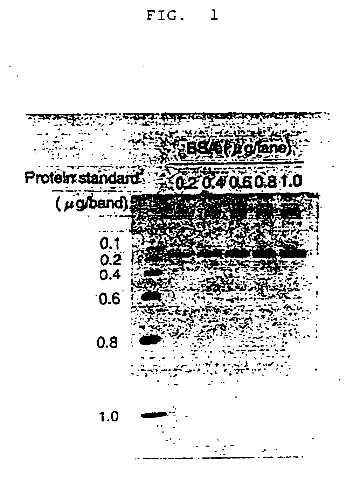

A marker consisting of pA(AB)₆ (M.W. 81,807), pA(AB)₅ (M. W. 68,406), pA(AB)₄ (M.W. 55,004), pA(AB)₃ (M.W. 41,603), pA(AB)₂ (M.W. 28,201) and pA(AB)₁ (M.W. 14,800) was dissolved in a buffer (62.5 mM Tril-HCl, pH 6.8, containing 2 % SDS, 5 % glycerol and 0.005 % Bromphenol Blue) to give the resultant buffer containing 0.1 µg, 0.2 µg, 0.4 µg, 0.6 µg, 0.8 µg and 1.0 µg, respectively, per 10 µl of the resultant buffer. Separetely, bovine serum albumin (BSA) was dissolved in the same buffer as above-mentioned to give the resultant buffers (5 buffers) each containing 0.2, 0.4, 0.6, 0.8 or 1.0 µg BSA per 10 µl of each resultant buffer. 10 µl of each of the obtained-resultant buffer of the marker and the 5 resultant buffers of BSA was subjected to SDS-polyacrylamide gel electrophoresis (SDS-PAGE) on a 15 to 25 % gradient gel and then stained with Coomassie Brilliant Blue (CBB). The stained gel is shown in FIG. 1.

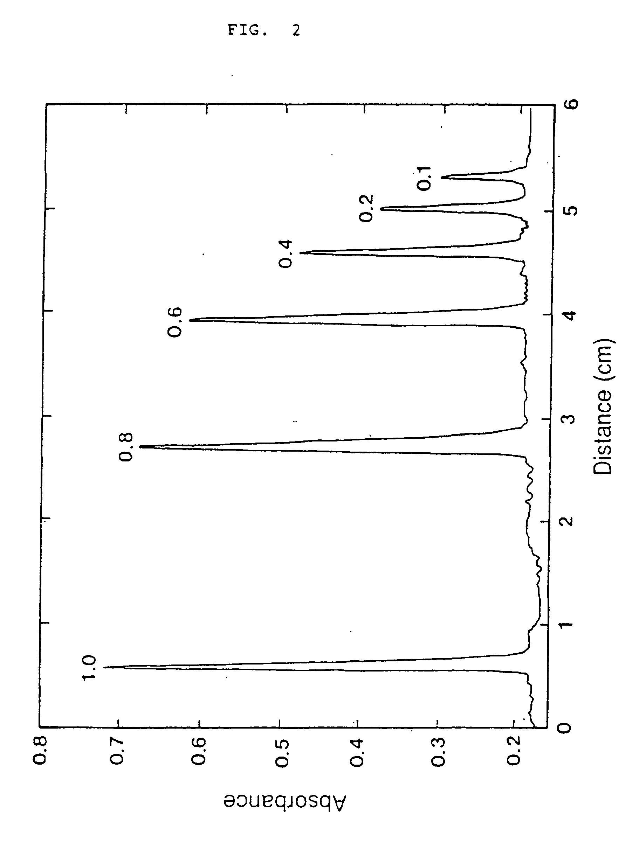

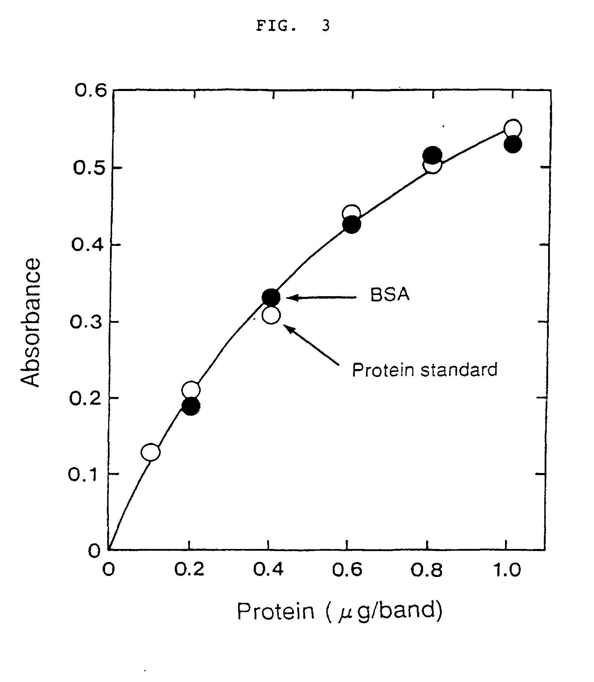

After staining, the gel was discolorized and dried, and the lane of the marker in the gel was scanned on an LKB ULTROSCAN XL Laser Densitometer to obtain a densitogram (FIG. 2), and a calibration curve was prepared using the density of each band (FIG. 3). As a result, the calibration curve of the protein standard almost agreed with that of BSA.

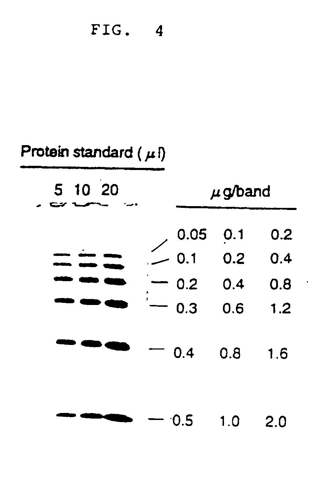

5, 10 and 20 µl of the above-mentioned resultant buffer of the marker were applied in the different lanes to SDS-PAGE and stained with CBB in the same manner as above. The result showed that a calibration curve could be obtained in the range of 0.05 to 2.0 µg/band (FIG. 4).

As is evident from these results, a protein in a sample can be determined for its molecular weight (or type: e.g. BSA) by use of the position of the band as well as for its amount (or concentration) by use of the density of the band.

That is, according to the present invention, not only the molecular weight or amount of each protein in an unknown sample, but also both the molecular weight and amount of each protein can be determined simultaneously by means of the marker such as the molecular weight marker.

### Effect of the Invention

According to the present invention, the molecular weight and protein amount desired can be regulated arbitrarily by selecting the type and mixing ratio of constituent marker proteins. The necessary data for preparing a calibration curve is obtained by electrophoresing the molecular weight marker in only one lane, and the amount of the charged protein is proportional to the density of its band, so the amount of the protein can be estimated approximately by visual examination or determined using the calibration curve.

Further, by electrophoresing an unknown protein-containing sample simultaneously with the marker such as the molecular weight marker, the protein in the unknown sample can be determined for its type (molecular weight) and its concentration (amount) simultaneously by visually examining the position and density of its stained band or with a densitometer for more accurate measurement.

The Sequence Listing, which follows is concerned with the N-terminal end (i.e. MKVD as SEQ. ID. No 1) and the C-terminal end (i.e. TGRRFTTS as SEQ. ID. No 2) of the marker proteins of Tables I and II.

## Claims

1. A method for determining the molecular weight and/or the amount of a target protein, which comprises
(1) subjecting a target protein to electrophoresis simultaneously with a marker consisting of proteins having different molecular weights, the respective amounts of the proteins of the marker being different from one another,
(2) staining the target protein and the proteins of the marker, and
(3) comparing the bands induced in step (2) from one another by eye or with a densitometer,
whereby the proteins of the marker are IgG-binding artificial proteins each comprising one or several iterations of a unit comprising one or several domains selected from the group consisting of domains A, B, C, D and E of protein A.

2. The method according to claim 1, wherein the unit comprises domains A and B of protein A.

3. The method according to claim 1 or 2, wherein the target protein is a single protein or a mixture of proteins.

## Patentansprüche

1. Verfahren zur Bestimmung des Molekulargewichts und/oder der Menge eines Zielproteins, umfassend
(1) das Unterwerfen eines Zielproteins einer Elektrophorese gleichzeitig mit einem Marker, der aus Proteinen mit verschiedenen Molekulargewichten besteht, wobei die jeweiligen Mengen der Proteine des Markers voneinander unterschiedlich sind,
(2) das Färben des Zielproteins und der Proteine des Markers, und
(3) das Miteinandervergleichen der in Schritt (2) hervorgerufenen Banden mit dem Auge oder mit einem Densitometer,
wobei die Proteine des Markers IgG-bindende, künstliche Proteine sind, wobei jedes eine oder mehrere Wiederholungen einer Einheit, umfassend ein oder mehrere Domänen, ausgewählt aus der Gruppe, bestehend aus den Domänen A, B, C, D und E des Proteins A, umfaßt.

2. Verfahren nach Anspruch 1, wobei die Einheit die Domänen A und B des Proteins A umfaßt.

3. Verfahren nach Anspruch 1 oder 2, wobei das Zielprotein ein einzelnes Protein oder ein Gemisch von Proteinen ist.

## Revendications

1. Méthode pour déterminer le poids moléculaire et/ou la quantité d'une protéine cible, comprenant les étapes consistant à :
(1) soumettre une protéine cible à l'électrophorèse simultanément avec un marqueur consistant en des protéines ayant des poids moléculaires différents, les quantités respectives des protéines du marqueur étant différentes les unes des autres,
(2) colorer la protéine cible et les protéines du marqueur et
(3) comparer les bandes induites à l'étape (2) les unes aux autres à l'oeil ou en utilisant un densitomètre,
les protéines du marqueur étant des protéines artificielles fixant les IgG chacune comprenant une ou plusieurs itérations d'une unité comprenant un ou plusieurs domaines sélectionnés dans le groupe comprenant les domaines A, B, C, D et E de la protéine A.

2. Méthode selon la revendication 1 dans laquelle l'unité comprend les domaines A et B de la protéine A.

3. Méthode selon la revendication 1 ou 2 dans laquelle la protéine cible est une seule protéine ou un mélange de protéines.
